(19) Europäisches Patentamt
European Patent Office
Office européen des brevets

(11) Publication number: **0 491 441 A1**

# (12) EUROPEAN PATENT APPLICATION

(21) Application number: **91203316.4**

(22) Date of filing: **16.12.91**

(51) Int. Cl.5: **C07D 217/16**, C07D 217/22, A01N 43/42, C07D 217/14, C07D 491/04, //(C07D491/04, 317:00,221:00)

(30) Priority: **17.12.90 EP 90124372**

(43) Date of publication of application: **24.06.92 Bulletin 92/26**

(84) Designated Contracting States: **AT BE CH DE DK ES FR GB GR IT LI LU NL**

(71) Applicant: **SHELL INTERNATIONALE RESEARCH MAATSCHAPPIJ B.V.**
**Carel van Bylandtlaan 30**
**NL-2596 HR Den Haag(NL)**

(72) Inventor: **Bissinger, Hans-Joachim**
**Leidsevaart 274**
**NL-2014 HH Haarlem(NL)**
Inventor: **Schröder, Ludwig**
**Frankenstrasse 7**
**W-6507 Ingelheim am Rhein(DE)**
Inventor: **Albert, Guido**
**Volxheimerstrasse 4**
**W-6551 Hackenheim(DE)**
Inventor: **Pees, Klaus-Jürgen**
**Soonwald Strasse 9**
**W-6500 Mainz(DE)**

## (54) Fungicidal isoquinoline derivatives.

(57) The invention provides a method of combating fungus at a locus which comprises treating the locus with a compound of the general formula

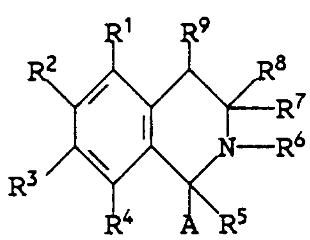

(I)

or an N-alkyl or N-phenyl halide or N-oxide thereof, in which $R^1$, $R^2$, $R^3$ and $R^4$ independently represent a hydrogen or halogen atom, a hydroxyl group or an optionally substituted alkyl or alkoxy group, or $R^1$ and $R^2$ or $R^2$ and $R^3$ or $R^3$ and $R^4$ together with the interjacent carbon atoms represent a 5- to 7-membered saturated or unsaturated carbocyclic or heterocyclic ring in which a heterocyclic ring contains 1 to 3 heteroatoms selected from nitrogen, oxygen and sulphur atoms; $R^5$ represents a hydrogen or halogen atom, $R^6$ represents a hydrogen atom, or $R^5$ and $R^6$ together represent a single carbon- carbon bond; $R^7$ represents a hydrogen or halogen atom, an alkyl or alkoxy group optionally substituted by one or more halogen atoms, or an optionally substituted phenyl or phenoxy group; $R^8$ and $R^9$ independently represent a hydrogen atom or together represent a single carbon-carbon bond; and A represents an optionally substituted phenyl group. Certain of the isoquinoline derivatives are novel and a process for the preparation of these compounds and compositions containing them are also provided.

EP 0 491 441 A1

The present invention relates to a method of combating fungi, especially phytopathogenic fungi, utilising certain isoquinoline derivatives, some of which are novel, a process for their preparation and compositions containing such compounds.

US 4717724 discloses compounds of the formula

$G\text{-}CH_2\text{-}O\text{-}D$ (A)

in which G represents a 2-aryl-2-(1H-imidazol-1-yl-methyl)-1,3-dioxolan-4-ylmethyloxy or 2-aryl-2-(1,2,4-triazol-1-ylmethyl)-1,3-dioxolan-4-ylmethyloxygroup and D represents an isoquinolin-6-yl, 3,4-dihydroisoquinolin-6-yl or 1,2,3,4-tetrahydroisoquinolin-6-yl group. These compounds are said to be chemotherapeutic agents with activity against certain skin fungi, mold fungi, yeasts and bacteria. However, there is no suggestion of any activity against phytopathogenic fungi.

EP 0170524 A2 discloses isoquinolines and 3,4-dihydroisoquinolines substituted at the 1-position by a group $-C(R^A)=C(R^BR^C)$ in which $R^A$ represents hydrogen or fluorine, $R^B$ represents hydrogen, halogen, cyano, nitro or $C_{1-2}$ alkyl optionally substituted by one or more halogen atoms, and $R^C$ represents a group $-(CH=CH)_nR^D$ where n is 0-2 and $R^D$ represents hydrogen, $C_{1-8}$ alkyl, $C_{2-8}$ alkenyl, $C_{2-8}$ alkynyl or a monocyclic, bicyclic, tricyclic or tetracyclic ring system containing between three and sixteen ring members, optionally substituted by hydroxy, thio, halogen, fluorosulphonyl, nitro, cyano, $C_{1-12}$ hydrocarbyl, $C_{1-12}$hydrocarbyloxy, $C_{1-12}$ hydrocarbylthio wherein the said hydrocarbyl, hydrocarbyloxy and hydrocarbylthio groups are each optionally further substituted by halogen, hydroxy, thio, $C_{1-2}$ alkylthio or $C_{1-2}$ alkoxy groups, the groups $R^B$ and $R^C$ optionally being linked through a $C_{1-4}$ alkylene bridge. These compounds are said to possess broad spectrum antifungal activity against fungi pathogenic in man, animals and plants. However, no evidence of activity against phytopathogenic fungi is provided.

EP 0251361 A1 discloses di- and tetrahydroisoquinoline derivatives substituted, inter alia, at the 1-position by an optionally substituted phenyl group and at the 3-position by substituents $R^E$ and $R^F$ whre $R^E$ is hydrogen or a group $-CO-OR^G$ in which $R^G$ is $C_{1-4}$ alkyl, or $R^E$ is a group $-CON(R^H)_2$ in which both groups $R^H$ independently represent hydrogen or $C_{1-4}$ alkyl, or $R^E$ is a group $-CH_2OR^J$ in which $R^J$ is hydrogen, $C_{1-4}$ alkyl, tetrahydropyranyl, $C_{1-4}$ alkanoyl, or carbamoyl optionally substituted by 1 or 2 $C_{1-4}$ alkyl groups, and $R^F$ is hydrogen or methyl. However, only one compound is specifically disclosed in which both $R^E$ and $R^F$ are hydrogen and that is 6,7-methylenedioxy-1-(3,4,5-trimethoxyphenyl)-tetrahydroisoquinoline. The compounds are described as cytostatic agents, that is, they have antitumour activity.

It has now been discovered that certain isoquinoline derivatives exhibit good activity against certain phytopathogenic fungi, particularly against fungi of the Erysiphe genus and, especially, powdery mildews in cereals.

According to the present invention there is therefore provided a method of combating fungus at a locus which comprises treating the locus with a compound of the general formula.

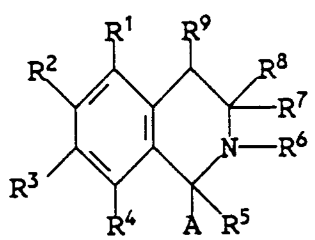

(I)

or an N-alkyl or N-phenyl halide or N-oxide thereof, in which

$R^1$, $R^2$, $R^3$ and $R^4$ independently represent a hydrogen or halogen atom, a hydroxyl group or an optionally substituted alkyl or alkoxy group, or $R^1$ and $R^2$ or $R^2$ and $R^3$ or $R^3$ and $R^4$ together with the interjacent carbon atoms represent a 5- to 7-membered saturated or unsaturated carbocyclic or heterocyclic ring in which a heterocyclic ring contains 1 to 3 heteroatoms selected from nitrogen, oxygen and sulphur atoms;

$R^5$ represents a hydrogen or halogen atom,

$R^6$ represents a hydrogen atom, or

$R^5$ and $R^6$ together represent a single carbon- carbon bond;

$R^7$ represents a hydrogen or halogen atom, an alkyl or alkoxy group optionally substituted by one or more halogen atoms or an optionally substituted phenyl or phenoxy group;

$R^8$ and $R^9$ independently represent a hydrogen atom or together represent a single carbon-carbon bond; and

A represents an optionally substituted phenyl group.

When the compounds of formula I contain an alkyl substituent group, this may be linear or branched and may contain up to 12, preferably up to 6, and especially up to 4, carbon atoms. An aralkyl group may be any alkyl group substituted by an aryl group, especially a benzyl group. A 5- to 7-membered saturated or unsaturated carbocyclic ring may be any 5- to 7-membered ring composed solely of carbon and hydrogen atoms, such as a cycloalkyl, cycloalkenyl, cycloalkynyl or cycloalkadienyl ring, with 5- and 6-membered rings being especially preferred. Examples of 5- to 7-membered saturated or unsaturated heterocyclic rings include tetrahydrofuran, furan, tetrahydrothiophene, thiophene, pyrrolidine, pyrrole, pyrroline, pyrazole, imidazole, triazole, pyrazoline, oxazole, thiazole, isoxazole, isothiazole, pyran, dihydropyran, tetrahydropyran, thiopyran, dihydrothiopyran, tetrahydrothiopyran, pyridine, piperidine, pyridazine, dihydropyridazine, tetrahydropyridazine, pyrimidine, dihydropyrimidine, tetrahydropyrimidine, pyrazine, dihydropyrazine, tetrahydropyrazine, morpholine, thiazine, dihydrothiazine, tetrahydrothiazine, piperazine and triazine.

When any of the foregoing substituents are designated as being optionally substituted, the substituent groups which are optionally present may be any one or more of those customarily employed in the development of pesticidal compounds and/or the modification of such compounds to influence their structure/activity, persistence, penetration or other property. Specific examples of such substituents include, for example, halogen atoms, nitro, cyano, hydroxyl, cycloalkyl, alkyl, haloalkyl, alkoxy, haloalkoxy, amino, alkylamino, dialkylamino, formyl, alkoxycarbonyl, carboxyl, alkanoyl, alkylthio, alkylsulphinyl, alkylsulphonyl, aralkyl, phenyl, phenoxy, pyridyl, carbamoyl and alkylamido groups. These optional substituents may themselves be optionally substituted, for instance, by one or more halogen atoms. When any of the foregoing substituents represents or contains an alkyl group, this may be linear or branched and may contain up to 12, preferably up to 6, and especially up to 4, carbon atoms.

It is preferred that $R^1$, $R^2$, $R^3$ and $R^4$ independently represent a hydrogen or halogen atom, a hydroxyl group or a $C_{1-6}$ alkyl or $C_{1-6}$ alkoxy group each optionally substituted by one or more halogen atoms or $C_{1-4}$ alkoxy groups, or $R^1$ and $R^2$ or $R^2$ and $R^3$ or $R^3$ and $R^4$ together with the interjacent carbon atoms represents a 5- or 6-membered saturated heterocyclic ring containing two oxygen atoms especially a dioxane or dioxolane ring.

It is also preferred that $R^7$ represents a hydrogen atom or a $C_{1-6}$ alkyl, especially $C_{1-4}$ alkyl, group.

Preferably, A represents a group

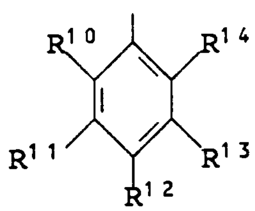

in which

$R^{10}$, $R^{11}$, $R^{12}$, $R^{13}$ and $R^{14}$ independently represent a hydrogen or halogen atom, a hydroxyl, nitro or amino group, or an optionally substituted alkyl, alkoxy, alkylamino, dialkylamino, alkylthio, alkylsulphinyl, alkylsulphonyl, aralkyl, phenyl, phenoxy or pyridyl group.

More preferably, $R^{10}$, $R^{11}$, $R^{12}$, $R^{13}$ and $R^{14}$ independently represent a hydrogen or halogen atom, a hydroxyl, nitro or amino group, or an alkyl, alkoxy, alkylsulphonyl, benzyl, phenyl, phenoxy or pyridyl group each optionally substituted by one or more halogen atoms.

A particularly preferred sub-group of compounds is that in which $R^1$, $R^2$, $R^3$ and $R^4$ independently represent a hydrogen, chlorine or bromine atom or a hydroxyl, methyl, ethyl, propyl, methoxy, ethoxy, propoxy, butoxy, pentyloxy, hexyloxy or methoxyethoxy group, or $R^2$ and $R^3$ together with the interjacent carbon atoms represent a 1,3-dioxolane ring; $R^5$ represents a hydrogen or chlorine atom, $R^6$ represents a hydrogen atom, or $R^5$ and $R^6$ together represent a single carbon-carbon bond; $R^7$ represents a hydrogen atom or an ethyl group; and $R^{10}$, $R^{11}$, $R^{12}$, $R^{13}$ and $R^{14}$ independently represent a hydrogen, fluorine, chlorine, bromine or iodine atom or a hydroxyl, nitro, amino, methyl, trifluoromethyl, methoxy, methylsulphonyl, benzyl, phenyl, phenoxy or chloropyridyl group.

Preferably, the locus comprises plants subject to or subjected to fungal attack, seeds of such plants or

the medium in which such plants are growing or are to be grown.

The present invention still further provides the use as a fungicide of a compound of the general formula I as defined above or an N-alkyl or N-phenyl halide or N-oxide thereof, preferably for the treatment of fungal diseases in plants. In particular, the compounds according to general formula I may be advantageously used for the treatment of powdery mildew in plants, especially for the control of fungi of the Erysiphe genus.

Certain of the compounds of formula I are novel and the invention therefore also provides a compound of the general formula I or an N-alkyl or N-phenyl halide or N-oxide thereof as defined above with the proviso that, when $R^1$, $R^4$, $R^5$, $R^6$, $R^7$, $R^8$ and $R^9$ all represent a hydrogen atom and A represents a 3,4,5-trimethoxyphenyl group, then $R^2$ and $R^3$ together with the interjacent carbon atoms do not represent a 1,3-dioxolane ring.

The present invention also provides a process for the preparation of a compound of formula I as defined in the preceding paragraph or an N-alkyl or N-phenyl halide or N-oxide thereof which comprises

(a) cyclising a compound of the general formula

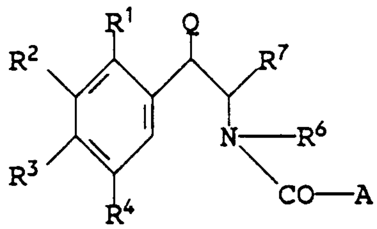

(II)

in which $R^1$, $R^2$, $R^3$, $R^4$, $R^6$, $R^7$ and A are as defined above and, when $R^8$ and $R^9$ in the resultant compound of formula I both represent a hydrogen atom then Q represents a hydrogen atom, and, when $R^8$ and $R^9$ in the resultant compound of formula I together represent a single carbon-carbon bond then Q represents an alkoxy, especially methoxy, group, in the presence of a dehydrating agent to form a compound of formula I in which $R^5$ and $R^6$ together represent a single carbon-carbon bond;

followed, if desired, when $R^8$ and $R^9$ both represent a hydrogen atom, by

(b) hydrogenating the resulting compound of formula I to produce a compound of formula I in which $R^5$, $R^6$, $R^8$ and $R^9$ all represent a hydrogen atom; or

(c) dehydrogenating the resulting compound of formula I to produce a compound of formula I in which $R^5$ and $R^6$ together and $R^8$ and $R^9$ together both represent a single carbon-carbon bond;

followed, if desired, by

(d) oxidising the compound of formula I formed in (a), (b) or (c) to form an N-oxide thereof; or

(e) converting the compound of formula I formed in (a), (b) or (c) to the corresponding N-alkyl or N-phenyl halide.

The dehydrating agent used in step (a) may be selected from phosphorus oxychloride, phosphorus pentoxide, phosphorus pentachloride, polyphosphoric acid, aluminium chloride and thionyl chloride with phosphorus oxychloride being particularly pareferred. Preferably, the reaction is carried out in an inert solvent, such as chloroform, benzene, toluene, xylene, nitrobenzene or tetraline. Depending on the reactivity of the reactants, the reaction may be carried out under cooling, at room temperature or at elevated temperature up to the boiling point of the reaction mixture. Generally, this step is suitably carried out under reflux.

Preferably, the hydrogenation in step (b) is accomplished by catalytic hydrogenation, that is, by using hydrogen gas under elevated pressure in the presence of a catalyst preferably selected from the platinum group of elements, such as platinum dioxide. This step is conveniently carried out at room temperature in the presence of an inert solvent, such as methanol or ethanol.

It is also preferred that the dehydrogenation in step (c) is accomplished by reaction with an oxidising agent, such as potassium permanganate used under acidic conditions or palladium on charcoal in the absence of hydrogen. This step may be conveniently carried out at a temperature from room temperature to the reflux temperature of the reaction mixture depending on the nature of the dehydrogenating agent used.

Preferably, the oxidising agent used in step (d) is a peracid, such as m-chloroperbenzoic acid. This step may be conveniently carried out at room temperature.

A compound of formula II in which Q represents a hydrogen atom may be conveniently prepared by the process of A. Bischler and B. Napieralski, Ber. dtsch. chem. Ges. 26, 1903 (1893) in which an amine of

general formula III

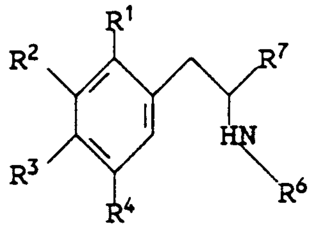

(III)

wherein $R^1$, $R^2$, $R^3$, $R^4$, $R^6$ and $R^7$ are as hereinbefore defined is reacted with an acid derivative of general formula A-CO-X or A-CO-O-CO-A in which A is as hereinbefore defined and X is a bromine or chlorine atom.

Accordingly, an appropriately substitued $\beta$-aryl ethylamine is reacted with a substituted benzoic acid derivative, such as the acid chloride or anhydride, in an inert solvent, e.g. benzene, toluene, dichloromethane, or a promoting solvent, e.g. pyridine, lutidine, or in mixtures thereof. When inert solvents are used it is advantageous to add at least one equivalent of a tertiary amine, such as triethylamine, diisopropyl ethylamine or 2,4,6-collidine. Depending on the reactivity of the reactants, the reaction may be carried out under cooling, at room temperature or at elevated temperature up to the boiling point of the reaction mixture. Generally, the reaction takes place under cooling.

A compound of formula II in which Q represents an alkoxy group may be prepared according to C.U.F. Mannich and M. Falber, Arch. Pharm., 267, 601 (1929).

Compounds of formula III and the acid derivatives of formula A-CO-X and A-CO-O-CO-A are known compounds or can be prepared by processes analogous to known processes.

The invention also provides fungicidal compositions which comprise a carrier and, as active ingredient, at least one of the compounds according to general formula I, as well as procedures for control of phytopathogenic fungi.

The compounds according to general formula I may be used as such, however, they are preferably used as compositions comprising, besides the compounds according to the invention, adjuvants and auxiliaries which are known for formulation purposes and may be manufactured into e.g. emulsion concentrates, solutions which may be sprayed directly or diluted, diluted emulsions, wettable powders, soluble powders, dusts, granulates, microencapsulates by well-established procedures. The form of application such as spraying, atomising, dispersing, pouring may be chosen, like the compositions, according to the desired objectives and the given circumstances.

The formulations, that is, the compositions which comprise at least one compound according to general formula I and optionally solid and/or liquid auxiliaries and adjuvants, may be prepared by well-established procedures, e.g. intensive mixing and/or grinding of the active ingredients with other substances, such as fillers, solvents, solid carriers, and optionally surface-active compounds (tensides). The composition may contain at least two carriers, at least one of which is a surface-active agent.

Solvents may be aromatic hydrocarbons, preferably the fractions $C_8$ to $C_{12}$, e.g. xylenes or xylene mixtures, substituted naphthalenes, phthalic acid esters, such as dibutyl or dioctyl phthalate, aliphatic hydrocarbons, e.g. cyclohexane or paraffins, alcohols and glycols as well as their ethers and esters, e.g. ethanol, ethyleneglycol mono- and dimethyl ether, ketones such as cyclohexanone, strongly polar solvents such as N-methyl 2-pyrrolidone, dimethyl sulphoxide, alkyl formamides, epoxidised vegetable oils, e.g. epoxidised coconut or soybean oil, water.

Solid carriers, which may be used for dusts or dispersible powders, may be mineral fillers, such as calcite, talc, kaolin, montmorillonite, attapulgite. The physical properties may be improved by addition of highly dispersed silica gel or highly dispersed polymers. Carriers for granulates may be porous material, e.g. pumice, broken brick, sepiolite, bentonite, non-sorptive carriers may be calcite or sand. Additionally, a multitide of pre-granulated inorganic or organic materials may be used, such as dolomite or crushed plant residues.

Suitable surface-active substances may be non-ionogenic, anionic or cationic tensides with good dispersing, emulgating and wetting properties depending on the nature of the compound according to general formula I to be formulated. Tensides may also mean mixtures of tensides.

Suitable tensides may be so-called water-soluble soaps as well as water-soluble synthetic surface-active compounds.

Soaps usually are alkali, earth alkali or optionally-substituted ammonium salts of higher fatty acids ($C_{10}$-$C_{20}$), e.g. the sodium or potassium salts of oleic or stearic acid or of mixtures of natural fatty acids which are prepared, for example, from coconut or tallow oil. Furthermore, methyl-taurin salts of fatty acids may be used.

However, so-called synthetic tensides are preferably used, especially fatty sulphonates, fatty sulphates, sulphonated benzimidazole derivatives or alkyl aryl sulphonates.

The fatty sulphates or fatty sulphonates are normally used as alkali, earth alkali or optionally-substituted ammonium salts and have an alkyl moiety of 8 to 22 carbon atoms, whereby alkyl also means the alkyl moiety of acyl residues, such as the sodium or calcium salt of lignin sulphonic acid, of sulphuric acid dodecylate or of a mixture of fatty alcohols prepared from natural fatty acids. This also includes the salts of sulphuric acid esters, sulphonic acids and adducts of fatty alcohols and ethylene oxide. The sulphonated benzimidazole derivatives preferably contain 2 sulphonic acid residues and a fatty acid residue with 8 to 22 carbon atoms. Alkyl aryl sulphonates are, for example, the sodium, calcium or triethyl ammonium salts of dodecyl benzene sulphonic acid, dibutyl naphthalene sulphonic acid or of a condensate of naphthalene sulphonic acid and formaldehyde.

Furthermore, phosphates, such as the salts of the phosphoric acid ester of a p-nonylphenol-(4-14)-ethylene oxide adduct or phospholipids, may be used.

Non-ionic tensides are preferably polyglycolether derivatives of aliphatic or cycloaliphatic alcohols, saturated or non-saturated fatty acids and alkylphenols, which have 3 to 10 glycol ether groups and 8 to 20 carbon atoms in the (aliphatic) hydrocarbon residue and 6 to 18 carbon atoms in the alkyl residue of the alkyl phenols.

Other suitable non-ionic tensides are the water-soluble, 20 to 250 ethylene glycol ether groups containing polyadducts of ethylene oxide and polypropylene glycol, ethylene diamino polypropylene glycol and alkyl polypropylene glycol with 1 to 10 carbon atoms in the alkyl moiety, the substances normally contain 1 to 5 ethylene glycol units per propylene glycol unit.

Examples of non-ionic tensides are nonylphenol polyethoxy ethanols, castor oil polyglycol ether, polyadducts of ethylene oxide and polypropylene, tributyl phenoxy polyethoxy ethanol, polyethylene glycol, octyl phenoxy polyethoxy ethanol.

Furthermore, fatty acid esters of polyoxy ethylene sorbitan, such as polyoxy ethylene sorbitan trioleate may be used.

Cationic tensides preferably are quarternary ammonium salts, which have at least one alkyl residue with 8 to 22 carbon atoms and, furthermore, low, optionally-halogenated alkyl, benzyl or hydroxyalkyl residues. The salts are preferably halides, methyl sulphates or alkyl sulphates, e.g. stearyl trimethyl ammonium chloride or benzyl bis(2-chloroethyl) ethyl ammonium bromide.

The tensides generally used for compositions are disclosed in such publications as:

"McCutheon's Detergents and Emulsifiers Annual", MC Publishing Corp., Ridgewood, NJ, USA 1981;

H. Stache, "Tensid-Taschenbuch", 2nd ed., C. Hanser, Munich, Vienna, 1981;

M. and J. Ash, "Encyclopedia of Surfactants", vol. I-III, Chemical Publishing Co., New York, NY, USA 1980-1981.

The pesticidal compositions usually comprise 0.1% to 95%, preferably 0.1% to 80% of at least one compound according to general formula I, 1% to 99.9% of a solid or liquid adjuvant and 0% to 25%, preferably 0.1% to 25%, of a tenside.

The preferred compositions usually comprise.

As commodity, the compositions may preferably be in a concentrated form whereas the end-user generally employs diluted compositions. The compositions may be diluted to a concentration of 0.001% of active ingredient (a.i.). The doses usually are in the range from 0.01 to 10 kg. a.i./ha.

The compositions may also comprise other auxiliaries such as stabilisers, defoamer, viscosity controlling agents, thickeners, adhesives, fertilisers or other active ingredients to obtain special effects.

The present invention is further illustrated by the following examples. It should be understood, however, that the invention is not limited solely to the particular examples given below.

## EXAMPLES

### EXAMPLE 1

Preparation of 1-(4'-phenoxyphenyl)-6,7-dimethoxy-3,4-dihydroisoquinoline
$R^1 = R^4 = H$; $R^2 = R^3 = CH_3O$; $R^5/R^6$ = single C-C bond; $R^7 = R^8 = R^9 = H$; A = 4-phenoxyphenyl)

(i) Preparation of N-[β-(3',4'-dimethoxyphenyl)ethyl]-4-phenoxy benzoic acid amide

β-(3,4-Dimethoxyphenyl)ethylamine (3.9g, 21.5mmol) and triethylamine (2ml) were dissolved in dichloromethane (50ml). 4-Phenoxy benzoic acid chloride (5g, 20mmol), dissolved in dichloromethane (10ml), was added over a period of 10 minutes with stirring whilst maintaining the temperature of the mixture below 10 °C. The stirring was continued for another 2 hours and the reaction mixture was then washed twice with water (50ml each), dried with magnesium sulphate and evaporated. The residue was triturated with diisopropyl ether and the precipitate collected by vacuum filtration and dried. Off-white crystals of N-[β-(3',4'-dimethoxyphenyl)ethyl]-4-phenoxy benzoic acid amide (7g, 88% of theoretical yield), m.pt. 118 °C, were obtained.

(ii) Preparation of 1-(4'-phenoxyphenyl)-6,7-dimethoxy-3,4-dihydroisoquinoline

The N-[β-(3',4'-dimethoxyphenyl)ethyl]-4-phenoxy benzoic acid amide (6.5g, 18.1mmol) prepared in (i) above was dissolved in phosphorus oxychloride (50ml) and heated for 10 hours under reflux. The phosphorous oxychloride was then removed by vacuum distillation and the residue dissolved in water. The solution was made alkaline by addition of 2N aqueous sodium hydroxide and extracted several times with dichloromethane. The collected organic layers were dried with magnesium sulphate and the solvent was then evaporated in vacuo. Subsequent column chromatography on silica gel with dichloromethane/acetone (5:1) as eluant yielded 4g, 1-(4'-phenoxyphenyl)-6,7-dimethoxy-3,4-dihydroisoquinoline (66% of theoretical yield) as a viscous oil.

$^1$H-NMR: (ppm) δ = 2.76(t,2H), 3.77(s,3H), 3.80 (t,2H), 3.98(s,3H), 6.82(s,1H), 6.87(s,1H), 7.11-(m,4H), 7.16 (t,1H), 7.41(t,2H), 7.62(d,2H)

## EXAMPLE 2

Preparation of 1-(2',6'-difluorophenyl)-6,7-dimethoxy-1,2,3,4-tetrahydroisoquinoline
($R^1 = R^4 = H$; $R^2 = R^3 = CH_3O$; $R^5 = R^6 = R^7 = R^8 = R^9 = H$; A = 2,6-difluorophenyl)

1-(2',6'-Difluorophenyl)-6,7-dimethoxy-3,4-dihydroisoquinoline (6.5g, 21.4mmol), prepared by a method analogous to Example 1, was dissolved in methanol. Platinum dioxide catalyst (0.7g) was added and the reaction mixture hydrogenated under a hydrogen pressure of 5bar for 3.5 hours at room temperature. The catalyst was then filtered off and the filtrate evaporated. The residue was applied onto a silica gel column and eluted with dichloromethane/acetone (10:1) to give 2g 1-(2',6'-difluorophenyl)-6,7-dimethoxy-1,2,3,4-tetrahydroisoquinoline (31% of theoretical; m.pt. 115 °C), which crystallised on evaporation.

$^1$H-NMR:(ppm) δ = 2.71(d,1H), 2.97(m,1H), 3.10 (m,1H), 3.40 (m,1H), 3.64(s,3H), 3.86(s,3H), 5.50-(s,1H), 6.18(s,1H), 6.60(s,1H), 6.90(t,2H), 7.25(m,1H)

## EXAMPLE 3

Preparation of 1-(2-nitrophenyl)-5,6-dimethoxyisoquinoline
($R^1 = R^2 = CH_3O$; $R^3 = R^4 = R^7 = H$; $R^5/R^6$ = single C-C bond; $R^7 = H$; $R^8/R^9$ = single C-C bond; A = 2-nitrophenyl)

N-(2-nitrobenzoyl)-2-methoxy-2-(2,3-dimethoxyphenyl)ethylamine (4.4g, 0.012mol) prepared according to C.U.F. Mannich and M. Falber, Arch. Pharm. 267, 601 (1929)) was dissolved in phosphorus oxychloride (20ml) and heated under reflux for 2 hours. After cooling down, the reaction mixture was poured into water (200ml), the solution was made alkaline (pH8) by addition of sodium hydroxide and extracted 2-3 times with ethyl acetate (100ml each). The organic layers were collected, dried with anhydrous sodium sulphate and the solvent was evaporated in vacuo. The residual dark oil was applied onto a silica gel column (15cm x 3.5cm). Elution with petrol ether/ethyl acetate (3:1) gave 2.76g 1-(2-nitrophenyl)-5,6-dimethoxyisoquinoline, which crystallized as yellowish crystals, m.pt. 95 °C, Yield: 73% of theoretical

$^1$H-NMR: δ = 4.01(s,3H), 4.02(s,3H), 7.35 (m,2H), 7.70(m,2H), 8.05(d,1H), 8.25(d,1H), 8.48ppm(d,1H)

## EXAMPLE 4

Preparation of 1-(2-fluorophenyl)-6,7-dimethoxyisoquinoline
($R^1 = R^4 = H$; $R^2 = R^3 = CH_3O$; $R^5/R^6$ = single C-C bond; $R^7 = H$; $R^8/R^9$ = single C-C bond; A = 2-fluorophenyl)

1-(2-Fluorophenyl)-6,7-dimethoxy-3,4-dihydroisoquinoline (5.7g, 0.019 mol; prepared according to N.W. Whaley et al., Organic Reactions VI, 74 (1951)) was dissolved in a mixture of water (100ml) and conc. HCl

and potassium permanganate (12.7g, 0.08mol) was added in several portions. The reaction mixture was stirred for another 4 hours, then the precipitated manganese dioxide was removed by filtration and the filter cake was washed with ethyl acetate (100ml). The filtrate was extracted three times with ethyl acetate (100ml each). All organic layers were collected, dried with anhydrous sodium sulphate and the solvent was evaporated in vacuo. The residual brownish oil was applied onto a silica gel column (15cm x 3.5cm) and eluted with petrol ether/ethyl acetate (3:1) to give 2.77g 1-(2-fluorophenyl)-6,7-dimethoxyisoquinoline as yellowish crystals, m.pt. 104° C, Yield: 40% of theoretical

$^1$H-NMR: $\delta$ = 3.83(s,3H), 4.04(s,3H), 7.00(s,1H), 7.35(m,6H), 8.45ppm (d,1H)

EXAMPLE 5

Preparation of 1-(2,6-difluorophenyl)-6,7-dimethoxyisoquinoline
$R^1 = R^4 = H$; $R^2 = R^3 = CH_3O$;$R^5/R^6$ = single C-C bond; $R^7 = H$; $R^8/R^9$ = single C-C bond; A = 2,6-difluorophenyl)

1-(2,6-Difluorophenyl)-6,7-dimethoxy-3,4-dihydroisoquinoline (6.5g, 0.021mol; prepared according to N.W. Whaley et al., Organic Reactions VI, 74 (1951)) was suspended in 60ml Decalin (Trade mark: decahydronaphthalene). Nitrobenzene (2.4g, 0.02mol) and palladium (10% on charcoal, 2.4g) were added. The reaction mixture was heated under reflux in a nitrogen atmosphere for 30 hours, then cooled down and the solvent evaporated in vacuo. The residue was taken up in dichloromethane (100ml) and filtered through silica gel. After evaporation of the solvent, the residue was applied onto a silica gel column (15cm x 3.5cm) and eluted with dichloromethane/acetone (10:1) to give 1.6g 1-(2,6-difluorophenyl)-6,7- dimethoxyisoquinoline as beige crystals, m.pt. 185° C. Yield: 25% of theoretical.

$^1$H-NMR: $\delta$ = 3.84(s,3H), 4.06(s,3H), 6.78 (s,1H), 7.09(m,3H), 7.44(m,1H), 7.62(d,1H), 8.55ppm (d,1H)

EXAMPLE 6

Preparation of 1-(2,6-Dichlorophenyl)-6-ethoxy-7-propoxyisoquinoline N-oxide:
$R^1 = R^4 = H$; $R^2 = C_2H_5O$; $R^3 = C_3H_7O$; $R^5/R^6$ single C-C bond; $R^7 = H$; $R^8/R^9$ = single C-C bond; A = 2,6-difluorophenyl;N-oxide)

1-(2,6-Dichlorophenyl)-6-ethoxy-7-propoxyisoquinoline (1.4g, 0.0037mol) prepared as described in any of Examples 3,4 and 5, and m-chloroperbenzoic acid (50%; 1.3g, 0.0037mol) in chloroform (20ml) were stirred at room temperatue for 2 hours. The reaction mixture was then washed with dilute aqueous sodium carbonate and with water (50ml each), the organic layer was dried with sodium sulphate and the solvent was evaporated in vacuo. The remaining reddish oil (1.7g) was applied onto a silica gel column (3.5 x 15cm). Elution with acetone/ethyl acetate (1:4) gave a yellow oil (1.4g) which crystallised upon trituration with petrol ether to give 1.2g 1-(2,6-dichlorophenyl)-6-ethoxy-7-propoxyisoquinoline N-oxide, m.pt. 148° C, Yield: 82% of theoretical

$^1$H-NMR:(ppm) $\delta$ = 1.00(t,3H); 1.56(t,3H); 1.74-1.82(m,2H); 3.82(m,2H); 4.23 (m,2H); 6.35(s,1H); 7.10-(s,1H); 7.37-7.66(m,4H); 8.30(d,1H)

EXAMPLE 7

Preparation of 1-(2-nitrophenyl)-3,4-dihydro-6-ethoxy-7-propoxy- isoquinoline N-oxide:
($R^1 = R^4 = H$;$R^2 = C_2H_5O$; $R^3 = C_3H_7O$; $R^5/R^6$ = single C-C- bond; $R^7 = R^8 = R^9 = H$; A = 2 = nitrophenyl; N-oxide)

1-(2-Nitrophenyl)-3,4-dihydro-6-ethoxy-7-propoxyisoquinoline (4.0g, 0.011mol), prepared as described in Example 1, and m-chloroperbenzoic acid (50%; 3.9g, 0.011mol) in chloroform (50ml) were stirred at room temperature for 2 hours. The reaction mixture was then washed with dilute aqueous sodium carbonate and with water (50ml each), the organic layer was dried with sodium sulphate and the solvent was evaporated in vacuo. The remaining orange-yellow oil (5.1g) was applied onto a silica gel column (3.5 x 15cm). Elution with acetone/ethyl acetate (1:4) gave 4.2g 1-(2-nitrophenyl)-3,4-dihydro-6-ethoxy-7-propoxyisoquinoline-N-oxide as orange-yellow crystals, m.pt. 82-83° C. Yield: 100% of theoretical.

$^1$H-NMR:(ppm) $\delta$ = 0.9(t,3H); 1.43(t,3H); 1.66(m,2H); 3.0-3.3(2m,2H); 4.1-4.2(m,2H); 6.2(s,1H); 6.25-(s,1H); 7.5-8.2(m,4H)

EXAMPLES 8 TO 119

By processes similar to those described in Examples 1 to 7 above, further compounds according to the invention were prepared as detailed in Table 1 below. In this table, the compounds are identified by reference to formula I. Physical data and NMR spectroscopic data for the compounds of Examples 8 to 119 are given in Table IA below.

TABLE I

| Example No. | $R^1$ | $R^2$ | $R^3$ | $R^4$ | $R^5$ $R^6$ | $R^7$ | $R^8$ | $R^9$ | A |
|---|---|---|---|---|---|---|---|---|---|
| 8 | H | H | $CH_3O$ | H | C-C bond | H | H | H | 2,6-$Cl_2$ Phenyl |
| 9 | H | H | H | H | " | " | " | " | " |
| 10 | H | $CH_3O$ | $CH_3O$ | H | " | " | " | " | 2,6-$F_2$ Phenyl |
| 11 | H | H | H | H | " | " | " | " | " |
| 12 | H | $CH_3O$ | $CH_3O$ | H | " | " | " | " | 2-I Phenyl |
| 13 | H | $CH_3O$ | $CH_3O$ | H | " | " | " | " | 2,6-$Cl_2$ Phenyl |
| 14 | H | H | $CH_3O$ | H | " | " | " | " | 2-I Phenyl |
| 15 | H | H | $CH_3O$ | H | " | " | " | " | 2,6-$F_2$ Phenyl |
| 16 | H | $CH_3O$ | $CH_3O$ | H | " | " | " | " | 2-F Phenyl |
| 17 | H | H | H | H | " | " | " | " | 2-I Phenyl |
| 18 | H | $CH_3O$ | $CH_3O$ | H | " | " | " | " | 2-Br Phenyl |
| 19 | H | Cl | H | H | " | " | " | " | 2-Cl Phenyl |
| 20 | H | H | $CH_3$ | H | " | " | " | " | " |
| 21 (N-methyl iodide) | H | $CH_3O$ | $CH_3O$ | H | " | " | " | " | 2,6 $Cl_2$ Phenyl |
| 22 | H | $CH_3O$ | $CH_3O$ | H | " | " | " | " | 2,6-$(CH_3O)_2$ Phenyl |

TABLE I (continued)

| Example No. | $R^1$ | $R^2$ | $R^3$ | $R^4$ | $R^5$ $R^6$ | $R^7$ | $R^8$ | $R^9$ | A |
|---|---|---|---|---|---|---|---|---|---|
| 23 | H | H | Br | H | C-C bond | H | H | H | 2-Cl Phenyl |
| 24 | H | $CH_3O$ | $CH_3O$ | H | " | " | " | " | 2-$CF_3$ Phenyl |
| 25 | H | H | H | $CH_3O$ | " | " | " | " | 2,6-$Cl_2$ Phenyl |
| 26 | H | $CH_3O$ | H | H | " | " | " | " | " |
| 27 | H | $CH_3O$ | $CH_3O$ | H | " | " | " | " | 2-(Phenyl)Phenyl |
| 28 | H | $CH_3O$ | $CH_3O$ | H | " | " | " | " | 2-(Phenoxy)Phenyl |
| 29 | H | $CH_3O$ | $CH_3O$ | H | " | $C_2H_5$ | " | " | 2,6-$Cl_2$ Phenyl |
| 30 | H | $CH_3O$ | $CH_3O$ | H | " | " | " | " | 2,6-$F_2$ Phenyl |
| 31 | H | $CH_3O$ | $CH_3O$ | H | " | H | " | " | 2-Cl Phenyl |
| 32 | H | $CH_3O$ | $CH_3O$ | H | " | " | " | " | 2-Cl,6-F Phenyl |
| 33 | H | $CH_3O$ | $CH_3O$ | H | " | " | " | " | 2,3,4-$(CH_3O)_3$-Phenyl |
| 34 | H | $CH_3O$ | $CH_3O$ | H | " | " | " | " | 2-Cl,5-Br Phenyl |
| 35 | H | $CH_3O$ | $CH_3O$ | H | " | " | " | " | 2-Cl,4-$NO_2$ Phenyl |
| 36 | H | $CH_3O$ | $CH_3O$ | H | " | " | " | " | 3-F Phenyl |
| 37 | H | H | H | H | " | " | " | " | 3-F Phenyl |
| 38 | H | $CH_3O$ | $CH_3O$ | H | " | " | " | " | 3-Br Phenyl |

TABLE I (continued)

| Example No. | $R^1$ | $R^2$ | $R^3$ | $R^4$ | $R^5$ | $R^6$ | $R^7$ | $R^8$ | $R^9$ | A |
|---|---|---|---|---|---|---|---|---|---|---|
| 39 | H | H | H | H | C-C bond | | H | H | H | 3-Br Phenyl |
| 40 | H | $CH_3O$ | $CH_3O$ | H | | " | " | " | " | 3,5-$Cl_2$ Phenyl |
| 41 | H | $CH_3O$ | $CH_3O$ | H | | " | " | " | " | 3-Cl Phenyl |
| 42 | H | H | H | H | | " | " | " | " | " |
| 43 | H | $CH_3O$ | $CH_3O$ | H | | " | " | " | " | 3-$CF_3$ Phenyl |
| 44 | H | H | $CH_3O$ | H | | " | " | " | " | 3-F Phenyl |
| 45 | H | $CH_3O$ | $CH_3O$ | H | | " | " | " | " | 4-$SO_2CH_3$ Phenyl |
| 46 | H | $CH_3O$ | $CH_3O$ | H | | " | " | " | " | 4-$NO_2$ Phenyl |
| 47 | H | $CH_3O$ | $CH_3O$ | H | | " | " | " | " | 4-$CH_3$ Phenyl |
| 48 | H | $CH_3O$ | $CH_3O$ | H | | " | " | " | " | 4-F Phenyl |
| 49 | $CH_3O$ | H | H | H | | " | " | " | " | " |
| 50 | H | H | $CH_3$ | H | | " | " | " | " | " |
| 51 | H | Cl | H | H | | " | " | " | " | " |
| 52 | H | $CH_3O$ | $CH_3O$ | H | | " | " | " | " | Phenyl |
| 53 | H | $CH_3O$ | $CH_3O$ | H | | " | $C_2H_5$ | " | " | 4-F Phenyl |
| 54 | H | $CH_3O$ | $CH_3O$ | H | Cl | H | H | " | " | 2-Cl Phenyl |
| 55 | H | $-OCH_2O-$ | | H | C-C bond | | " | " | " | 2,6-$F_2$ Phenyl |

TABLE I (continued)

| Example No. | $R^1$ | $R^2$ | $R^3$ | $R^4$ | $R^5$ $R^6$ | $R^7$ | $R^8$ | $R^9$ | A |
|---|---|---|---|---|---|---|---|---|---|
| 56 | H | $CH_3O$ | $CH_3O$ | H | C-C bond | H | H | H | 2-OH Phenyl |
| 57 | H | $-OCH_2O-$ | | H | " | " | " | " | 2,6-$Cl_2$ Phenyl |
| 58 | H | $CH_3O$ | $C_2H_5O$ | H | " | " | " | " | " |
| 59 | H | $CH_3O$ | $CH_3O$ | H | " | " | " | " | 2-$CH_3O$ Phenyl |
| 60 | H | $CH_3O$ | $CH_3O$ | H | " | " | " | " | 2-$NO_2$ Phenyl |
| 61 | H | $CH_3O$ | OH | H | " | " | " | " | 2,6-$Cl_2$ Phenyl |
| 62 | H | OH | $CH_3O$ | H | " | " | " | " | " |
| 63 | H | $CH_3O$ | OH | H | " | " | " | " | 2-$CH_3$ Phenyl |
| 64 | H | $CH_3O$ | $CH_3O$ | H | " | " | " | " | 2-$NH_2$ Phenyl |
| 65 | H | $C_3H_7O$ | $CH_3O$ | H | " | " | " | " | 2,6-$Cl_2$ Phenyl |
| 66 | H | $C_3H_7O$ | $CH_3O$ | H | " | " | " | " | 2,6-$F_2$ Phenyl |
| 67 | H | $CH_3O$ | $C_2H_5O$ | H | " | " | " | " | " |
| 68 | H | $C_2H_5O$ | $CH_3O$ | H | " | " | " | " | 2,6-$Cl_2$ Phenyl |
| 69 | H | $C_2H_5O$ | $CH_3O$ | H | " | " | " | " | 2,6-$F_2$ Phenyl |
| 70 | H | $CH_3O$ | $(CH_3)_2CHO$ | H | " | " | " | " | 2,6-$Cl_2$ Phenyl |
| 71 | H | $CH_3O$ | $(CH_3)_2CHO$ | H | " | " | " | " | 2,6-$F_2$ Phenyl |
| 72 | H | $CH_3O$ | $C_3H_7O$ | H | " | " | " | " | 2,6-$Cl_2$ Phenyl |

TABLE I (continued)

| Example No. | $R^1$ | $R^2$ | $R^3$ | $R^4$ | $R^5$ | $R^6$ | $R^7$ | $R^8$ | $R^9$ | A |
|---|---|---|---|---|---|---|---|---|---|---|
| 73 | H | $CH_3O$ | $C_3H_7O$ | H | C-C bond | | H | H | H | 2,6-$F_2$ Phenyl |
| 74 | H | $CH_3O$ | $C_4H_9O$ | H | " | | " | " | " | 2,6-$Cl_2$ Phenyl |
| 75 | H | $CH_3O$ | $C_4H_9O$ | H | " | | " | " | " | 2,6-$F_2$ Phenyl |
| 76 | H | $CH_3O$ | $(CH_3)_2CHCH_2O$ | H | " | | " | " | " | 2,6-$Cl_2$ Phenyl |
| 77 | H | $CH_3O$ | $C_6H_{11}O$ | H | " | | " | " | " | 2,6-$Cl_2$ Phenyl |
| 78 | H | $CH_3O$ | $(CH_3)_2CHCH_2CH_2O$ | H | " | | " | " | " | " |
| 79 | H | $CH_3O$ | $CH_3OCH_2CH_2O$ | H | " | | " | " | " | " |
| 80 | $CH_3O$ | $CH_3O$ | H | H | " | | " | C-C bond | | 2-Cl Phenyl |
| 81 | $CH_3O$ | $CH_3O$ | H | H | " | | " | " | | 2-$NH_2$ Phenyl |
| 82 | $CH_3O$ | $CH_3O$ | H | H | " | | " | " | | 2-$CF_3$ Phenyl |
| 83 | $CH_3O$ | $CH_3O$ | H | H | " | | " | " | | 2,6-$(CH_3O)_2$ Phenyl |
| 84 | $CH_3O$ | $CH_3O$ | H | H | " | | " | " | | 2,3-$(CH_3O)_2$ Phenyl |
| 85 | $CH_3O$ | $CH_3O$ | H | H | " | | " | " | | 2-Cl,4-$NO_2$ Phenyl |
| 86 | $CH_3O$ | $CH_3O$ | H | H | " | | " | " | | 2-$CH_3$,3-$NO_2$ Phenyl |
| 87 | $CH_3O$ | $CH_3O$ | H | H | " | | " | " | | 2-$CH_3O$ Phenyl |
| 88 | $CH_3O$ | $CH_3O$ | H | H | " | | " | " | | 2,4-$F_2$,3,5-$Cl_2$ Phenyl |

TABLE I (continued)

| Example No. | $R^1$ | $R^2$ | $R^3$ | $R^4$ | $R^5$ $R^6$ | $R^7$ | $R^8$ $R^9$ | A |
|---|---|---|---|---|---|---|---|---|
| 89 | $CH_3O$ | $CH_3O$ | H | H | C-C bond | H | C-C bond | 2-$CH_3$ Phenyl |
| 90 | $CH_3O$ | $CH_3O$ | H | H | " | " | " | 2-Br Phenyl |
| 91 | $CH_3O$ | $CH_3O$ | H | H | " | " | " | 2-F Phenyl |
| 92 | $CH_3O$ | $CH_3O$ | H | H | " | " | " | 2-(2-Cl Pyrid-3-yl) Phenyl |
| 93 | $CH_3O$ | $CH_3O$ | H | H | " | " | " | 2-Benzyl Phenyl |
| 94 | $CH_3O$ | $CH_3O$ | H | H | " | " | " | 2,6-$F_2$ Phenyl |
| 95 | $CH_3O$ | $CH_3O$ | H | H | " | " | " | 2,6-$Cl_2$ Phenyl |
| 96 | H | $CH_3O$ | $CH_3O$ | H | " | " | " | 2,6-$Cl_2$ Phenyl |
| 97 | H | $CH_3O$ | $CH_3O$ | H | " | " | " | 2,4-$F_2$,3,5-$Cl_2$ Phenyl |
| 98 | H | $CH_3O$ | $CH_3O$ | H | " | " | " | 2-$CH_3$ Phenyl |
| 99 | H | $CH_3O$ | $CH_3O$ | H | " | " | " | 2-Cl Phenyl |
| 100 | H | $CH_3O$ | $CH_3O$ | H | " | " | " | 2,6-$(CH_3O)_2$ Phenyl |
| 101 | H | $CH_3O$ | $CH_3O$ | H | " | " | " | 2-$NO_2$ Phenyl |
| 102 | H | $CH_3O$ | $CH_3O$ | H | " | " | " | 2-$CF_3$ Phenyl |
| 103 | H | $CH_3O$ | $CH_3O$ | H | " | " | " | 2,4-$Cl_2$ Phenyl |

TABLE I (continued)

| Example No. | $R^1$ | $R^2$ | $R^3$ | $R^4$ | $R^5$ | $R^6$ | $R^7$ | $R^8$ | $R^9$ | A |
|---|---|---|---|---|---|---|---|---|---|---|
| 104 | H | $CH_3O$ | $CH_3O$ | H | C-C bond | | H | C-C bond | | 2-(2-Cl Pyrid-3-yl) Phenyl |
| 105 | H | $CH_3O$ | H | $CH_3O$ | " | | " | " | | 2,5-$Cl_2$,6-$CH_3O$ Phenyl |
| 106 | H | $CH_3O$ | H | $CH_3O$ | " | | " | " | | 2-$NO_2$ Phenyl |
| 107 | H | $CH_3O$ | H | $CH_3O$ | " | | " | " | | 2-F Phenyl |
| 108 | H | $CH_3O$ | H | $CH_3O$ | " | | " | " | | 2,4-$F_2$,3,5-$Cl_2$ Phenyl |
| 109 | H | $CH_3O$ | H | $CH_3O$ | " | | " | " | | 2-$CH_3$ Phenyl |
| 110 | H | $CH_3O$ | H | $CH_3O$ | " | | " | " | | 2,6-$F_2$ Phenyl |
| 111 | H | $C_2H_5$ | $C_3H_7$ | H | " | | " | " | | 2,6-$Cl_2$ Phenyl |
| 112 | H | $C_2H_5$ | $C_3H_7$ | H | " | | " | " | | 2-$NO_2$ Phenyl |
| 113 | H | $CH_3O$ | $C_2H_5O$ | H | " | | " | " | | 2,6-$Cl_2$ Phenyl |
| 114 (N-oxide) | H | $CH_3O$ | $CH_3O$ | H | " | | " | H | H | 2,4-$Cl_2$ Phenyl |
| 115 (N-oxide) | H | $CH_3O$ | $CH_3O$ | H | " | | " | " | " | 2-F,6-$CF_3$ Phenyl |
| 116 (N-oxide) | H | $CH_3O$ | $CH_3O$ | H | " | | " | " | " | 2,6-$Cl_2$ Phenyl |

TABLE I (continued)

| Example No. | $R^1$ | $R^2$ | $R^3$ | $R^4$ | $R^5$ | $R^6$ | $R^7$ | $R^8$ | $R^9$ | A |
|---|---|---|---|---|---|---|---|---|---|---|
| 117 (N-oxide) | H | $CH_3O$ | $CH_3O$ | H | C-C bond | | H | C-C bond | | 2,6-$Cl_2$ Phenyl |
| 118 (N-oxide) | H | $C_2H_5O$ | $C_3H_7O$ | H | " | | " | H | H | " |
| 119 (N-oxide) | H | $C_2H_5O$ | $C_3H_7O$ | H | " | | " | C-C bond | | 2-$NO_2$ Phenyl |

TABLE IA

| Ex. No. | M.pt. (°C) | $^1$H-NMR [ppm] |
|---|---|---|
| 8 | 80 | 2.88(t,2H), 3.67(m,2H), 3.74(s,3H), 3.80(s,3H), 6.84(d,2H), 7.16-7.38 (m,4H) |
| 9 | 95-98 | 2.90(m,2H), 3.97(m,2H), 6.89(d,1H), 7.17-7.43(m,6H) |
| 10 | 135 | 2.81(t,2H), 3.72(s,3H), 3.96(m,5H), 6.52(s,1H), 6.77(s,1H), 7.00(t,2H), 7.38(m,1H) |
| 11 | oil | |
| 12 | oil | 2.83(m,2H), 3.69(s,3H), 3.97(m,5H), 6.41(s,1H), 6.82(s,1H), 7.15(t,1H), 7.42(d,1H), 7.48(t,1H), 7.93(d,1H) |
| 13 | 96 | 2.87(t,2H), 3.20(s,3H), 3.93(s,3H), 3.98(t,2H), 6.36(s,1H), 6.76(s,1H), 7.30(t,1H), 7.38(d,2H) |
| 14 | oil | 2.81(t,2H), 3.72(s,3H), 3.82(t,2H), 6.42(d,1H), 6.93(dd,1H),7.10(t,1H), 7.18(d,1H), 7.37(t,1H), 7.42(t,1H), 7.86(d,1H) |
| 15 | oil | |
| 16 | 124 | 2.79(t,2H), 3.72(s,3H), 3.88(t,2H), 3.95(s,3H), 6.60(s,1H), 6.77(s;1H), 7.14(t,1H), 7.23(t,1H), 7.43(m,1H), 7.52(t,1H) |
| 17 | 108 | 2.85(t,2H), 3.95(m,2H), 6.86(d,1H), 7.05-7.30(m,3H), 7.35-7.50(m,3H), 7.89(d,1H) |
| 18 | 100 | 2.92(m,2H), 3.63(s,3H), 3.97(m,5H), 6.40(s,1H), 6.81(s,1H), 7.30-7.50 (m,3H), 7.67(d,1H) |
| 19 | oil | 2.89(m,2H), 3.80(m,2H), 6.83(d,1H), 7.17(dd,1H), 7.27(s,1H), 7.33-7.49 (m,4H) |

TABLE IA (continued)

| Ex. No. | M.pt. (°C) | $^1$H-NMR [ppm] |
|---|---|---|
| 20 | 87 | 2.23(s,3H), 2.82(m,2H), 3.90(m,2H), 6.74(s,1H), 7.18(m,1H), 7.35-7.45 (m,5H) |
| 21 | semi-crystal-line oil | |
| 22 | 140 | 2.17(s,2H), 2.81(t,2H), 3.64(s,3H), 3.72(s,6H), 3.91(s,3H), 6.46(s,1H), 6.62(d,2H), 6.75(s,1H), 7.30(t,1H) |
| 23 | | 2.89(m,2H), 3.90(m,2H), 6.49(d,1H), 7.15-7.50(m,6H) |
| 24 | oil | 2.80(t,2H), 3.62(s,3H), 3.90(m,5H), 6.32(s,1H), 6.77(s,1H), 7.43(d,1H), 7.58(m,2H), 7.74(d,1H) |
| 25 | 169 | 2.81(t,2H), 3.42(s,3H), 3.90(t,2H), 6.76(d,1H), 6.89(d,1H), 7.20(dd,1H), 7.33(m,3H) |
| 26 | 96 | 2.94(t,2H), 3.83(s,3H), 3.96(t,2H), 6.71(dd,1H), 6.82 (m,2H), 7.29 (dd,1H), 7.37(s,1H), 7.40(d,1H) |
| 27 | 145 | 1.86(m,2H), 2.66(m,2H), 3.60(s,3H), 3.84(s,3H), 6.29(s,1H), 6.55(s,1H), 7.14(m,3H), 7.32(m,2H), 7.50(m,4H) |
| 28 | oil | 2.43(m,2H), 3.76(s,3H), 3.96(m,5H), 6.65(m,4H), 6.96(t,1H), 7.03(d,1H), 7.18(t,2H), 7.29(t,1H), 7.43(t,1H), 7.56(d,1H) |
| 29 | 116 | 1.09(t,3H), 1.70(m,2H), 1.96(m,1H), 2.70(dd,1H), 2.99(dd,1H), 3.67 (s,3H), 3.95(s,3H), 6.37(s,1H), 6.75(s,1H), 7.29(m,1H), 7.37(m,2H) |
| 30 | 129 | 1.09(t,3H), 1.76(m,2H), 1.97(m,1H), 2.68(dd,1H), 2.94(dd,1H), 3.69(s,3H), 3.96(s,3H), 6.50(s,1H), 6.77(s,1H), 6.95(dd,2H), 7.38(m,1H) |

TABLE IA (continued)

| Ex. No. | M.pt. (°C) | $^1$H-NMR [ppm] |
|---|---|---|
| 31 | 104 | 2.81(m,2H), 3.66(s,3H), 3.95(s,3H), 4.02(m,2H), 6.42(s,1H), 6.77(s,1H), 7.41(m,4H) |
| 32 | 110 | 2.84(t,2H), 3.68(s,3H), 3.96(m,5H), 6.43(s,1H), 6.77(s,1H), 7.12(t,1H), 7.24(m,2H) |
| 33 | 93 | 2.81(t,2H), 3.70(s,3H), 3.71(s,3H), 3.88(s,3H), 3.95(s,3H), 3.97(s,3H), 6.58(s,1H), 6.74(d+s,2H), 7.10(d,1H) |
| 34 | 210 (de-comp.) | 3.23(m,2H), 3.74(s,3H), 4.04(s,3H), 4.23(m,2H), 6.56(s,1H), 6.97(s,1H), 7.44(d,1H), 7.72(dd,1H), 7.80(d,1H) |
| 35 | 190 | 2.83(m,2H), 3.66(s,3H), 3.97(s,3H), 4.05(m,2H), 6.32(s,1H), 6.78(s,1H), 7.65(d,1H), 8.22(dd,1H), 8.33(dd,1H) |
| 36 | 128 | 2.78(t,2H), 3.77(s,3H), 3.82(m,2H), 3.97(s,3H), 6.80(d,2H), 7.14(m,1H), 7.40(m,3H) |
| 37 | oil | 2.82(t,2H), 3.86(m,2H), 7.16(m,1H), 7.20-7.50(m,7H) |
| 38 | 94 | 2.74(t,2H), 3.76(s,3H), 3.83(t,2H), 3.98(s,3H), 6.76(s,1H), 6.79(s,1H), 7.32(t,1H), 7.55(2d,2H), 7.81(s,1H) |
| 39 | oil | 2.92(t,2H), 3.94(t,2H), 7.28(m,3H), 7.38(t,1H), 7.55(t,3H), 7.80(s,1H) |
| 40 | 135 | 2.76(t,2H), 3.78(s,3H), 3.83(m,2H), 3.96(s,3H), 6.73(s,1H), 6.81(s,1H), 7.44(s,1H),7.55(s,2H) |
| 41 | 112-114 | 2.76(t,2H), 3.76(s,3H), 3.83(m,2H), 3.96(s,3H), 6.75(s,1H), 6.88(s,1H), 7.32-7.51(m,3H), 7.64(s,1H) |
| 42 | 100-103 | |

TABLE IA (continued)

| Ex. No. | M.pt. (°C) | $^1$H-NMR [ppm] |
|---|---|---|
| 43 | oil | 2.79(t,2H), 3.74(s,3H), 3.88(t,2H), 3.97(s,3H), 6.74(s,1H), 6.82(s,1H), 7.58(t,1H), 7.71(d,1H), 7.83(d,1H), 7.92(s,1H) |
| 44 | oil | 2.77(t,2H), 3.75(s,3H), 3.85(t,2H), 6.82(d,1H), 6.98(dd,1H), 7.16(m,1H), 7.20(d,1H), 7.36(d,1H) |
| 45 | 172 | 2.78(t,2H), 3.10(s,3H), 3.76(s,3H), 3.84(t,2H), 3.97(s,3H), 6.66(s,1H), 6.82(s,1H), 7.83(d,2H), 8.04(d,2H) |
| 46 | 156 | 2.75(t,2H), 3.74(s,3H), 3.86(t,2H), 3.98(s,3H), 6.64(s,1H), 6.83(s,1H), 7.81(d,2H), 8.31(d,2H) |
| 47 | 131 | 2.41(s,3H),2.74(t,2H), 3.76(s,3H), 3.80(m,2H), 3.96(s,3H), 6.78(s,1H), 6.83(s,1H), 7.22(d,2H), 7.51(d,2H) |
| 48 | 125 | 2.76(t,2H), 3.76(s,3H), 3.81(m,2H), 3.96(s,3H), 6.77(s,1H), 6.79(s,1H), 7.10(t,2H), 7.62(dt,2H) |
| 49 | 77 | 2.78(t,2H), 3.41(t,2H), 3.90(s,3H), 6.85(d,1H), 7.00(d,1H), 7.09(t,1H), 7.22(t,1H), 7.59(t,2H) |
| 50 | oil | 2.31(s,3H), 2.78(t,2H), 3.83(t,2H), 7.02-7.25(m,5H), 7.61(m,2H) |
| 51 | semi-crystalline oil | 2.80(t,2H), 3.83(t, 2H), 7.05-7.35 (m,5H), 7.59(m,2H) |
| 52 | 92 | 2.76(t,2H), 3.76(s,3H), 3.83(t,2H), 3.97(s,3H), 6.78(s,1H), 6.80(s,1H), 7.44(m,3H), 7.60(dd,2H) |
| 53 | 119 | 1.14(t,3H), 1.70, 1.91(2xm,2H), 2.54 (dd,1H), 2.76(dd,1H), 3.38(m,1H), 3.75(s,3H), 3.96(s,3H), 6.74(s,1H), 6.79(s,1H), 7.12(t,2H), 7.61(m,2H) |

TABLE IA (continued)

| Ex. No. | M.pt. (°C) | $^{1}$H-NMR [ppm] |
|---|---|---|
| 54 | oil | 2.86(m,2H), 3.03(m,2H), 3.70(s,3H), 3.89(s,3H), 5.58(s,1H), 6.26(s,1H), 6.65(s,1H), 6.96(dd,1H), 7.17(m,2H), 7.42(d,1H) |
| 55 | 68 | 2.78(t,2H), 3.92(t,2H), 5.93(s,2H), 6.44(s,1H), 6.70(s,1H), 6.95(t,2H), 7.31(m,1H) |
| 56 | 105 | 2.70(t,2H), 3.73(t,2H), 3.86(s,3H), 3.96(s,3H), 6.82(m,2H), 7.07(d,1H), 7.14(s,1H), 7.32(m,1H), 7.58(d,1H), 14.50(br s,1H) |
| 57 | 108 | 2.78(t,2H), 3.92(t,2H), 5.93(s,2H), 6.31(s,1H), 6.74(s,1H), 7.27(t,1H), 7.31(m,1H) |
| 58 | 92 | 1.31(t,3H), 2.33(t,2H), 3.47(q,2H), 3.90(s,3H), 6.35(s,1H); 6.74(s,1H), 7.30(d,1H), 7.37(d,2H) |
| 59 | 100 | |
| 60 | 127 | |
| 61 | 234 | |
| 62 | 201 | |
| 63 | 142 | |
| 64 | 160 | |
| 65 | 105 | |
| 66 | 102 | |
| 67 | 91 | 1.38(t,3H), 2.84(t,2H), 3.95(m,5H), 6.56(s,1H), 6.77(s,1H), 7.01(t,2H), 7.38(m,1H) |
| 68 | 99 | |

TABLE IA (continued)

| Ex. No. | M.pt. (°C) | $^1$H-NMR [ppm] |
|---|---|---|
| 69 | 90 | |
| 70 | 126 | 1.19(d,6H), 2.33(t,2H), 3.88(s,3H) 3.95(m,2H), 4.23(septet,1H), 6.39 (s,1H), 6.76(s,1H), 7.28(t,1H), 7.36 (d,2H) |
| 71 | 123 | 1.23(d,6H), 2.80(t,2H), 3.88(s,3H), 3.92(t,2H), 4.25(septet,1H), 6.55(s,1H), 6.74(s,1H), 6.97(t,2H), 7.36(m,1H) |
| 72 | 69 | 0.91(t,3H), 1.72(sextet,2H), 2.84 (t,2H), 3.74(t,2H), 3.90(s,3H), 3.94 (t,2H), 6.36(s,1H), 6.76(s,1H), 7.27(m,1H), 7.36(d,2H) |
| 73 | 82 | 0.93(t,3H), 1.72(sextet,2H), 2.82 (t,2H), 3.76(t,2H), 3.90(s,3H), 3.94 (t,2H), 6.50(s,1H), 6.73(s,1H), 6.99 (t,2H), 7.37(m,1H) |
| 74 | oil | 0.80(t,3H), 1.31(dq,2H), 1.63 (quintet,2H), 2.78(t,2H), 3.76(t,2H), 3.84(s,3H), 3.88(dd,2H), 6.30(s,1H), 6.71(s,1H), 7.22(t,1H), 7.32(d,2H) |
| 75 | 80 | 0.88(t,3H), 1.37(dt,2H), 1.68(m,2H), 2.80(t,2H), 3.81(t,2H), 3.91(s,3H), 3.93(t,2H), 6.49(s,1H), 6.73(s,1H), 6.97(t,2H), 7.36(m,1H) |
| 76 | oil | 0.82(d,6H), 1.91(m,1H), 2.73(t,2H), 3.44(d,2H), 3.78(s,3H), 3.84(t,2H), 6.24(s,1H), 6.66(s,1H), 7.28(d,1H), 7.27(d,2H) |
| 77 | oil | 0.83(m,3H), 1.33(m,6H), 1.67(t,2H), 2.82(t,2H), 3.76(t,2H), 3.88(s,3H), 3.94(t,2H), 6.32(s,1H), 6.74(s,1H), 7.26(m,1H), 7.36(dd,2H) |

TABLE IA (continued)

| Ex. No. | M.pt. (°C) | $^{1}$H-NMR [ppm] |
|---|---|---|
| 78 | oil | 0.81(d,6H), 1.57(quintet,2H), 1.64 (m,1H), 2.82(t,2H), 3.82(t,2H), 3.89 (s,3H), 3.92(m,2H), 6.34(s,1H), 6.74 (s,1H), 7.28(d,1H), 7.37(d,2H) |
| 79 | 108 | 2.81(t,2H), 3.31(s,3H), 3.65(t,2H), 3.88(s,3H), 3.94(m,4H), 6.44(s,1H), 6.73(s,1H), 7.26(dd,1H), 7.35(d,2H) |
| 80 | 83 | 4.00(s,3H), 4.01(s,3H), 7.35(m,6H), 7.94(d,1H), 8.55(d,1H) |
| 81 | 85 (dec.) | 4.02(s,6H), 6.82(d,2H), 7.27(m,3H), 7.77(d,1H), 7.88(d,1H), 8.50(d,1H) |
| 82 | 114 | 4.00(s,3H), 4.02(s,3H), 7.24(m,2H), 7.40(d,1H), 7.62(m,2H), 7.32(dd,1H), 7.92(d,1H), 8.48(d,1H) |
| 83 | 83 | 3.64(s,6H), 3.96(s,3H), 4.00(s,3H), 6.70(d,2H), 7.20(m,1H), 7.38(m,2H), 7.90(d,1H), 8.58(d,1H) |
| 84 | 76 | 3.42(s,3H), 3.95(s,3H), 4.00(ds,6H), 7.02(m,2H), 7.24(m,2H), 7.54(dd,1H), 7.94(d,1H), 8.56(d,1H) |
| 85 | 111 | 4.09(ds,6H), 7.30(s,2H), 7.65(d,1H), 8.05(d,1H), 8.28(dd,1H), 8.44(d,1H), 8.48(d,1H) |
| 86 | 90-91 | 2.18(s,3H), 4.02(ds,6H), 7.25(m,2H), 7.48(m,2H), 7.95(m,2H), 8.56(d,1H) |
| 87 | 120 | 3.71(s,3H), 4.00(ds,6H), 7.05(m,2H), 7.20(m,1H), 7.44(m,3H), 7.88(d,1H), 8.52(d,1H) |
| 88 | 173-174 | 4.03(ds,6H), 7.35(d,1H), 7.55(m,2H), 7.98(d,1H), 8.55(d,1H) |
| 89 | 74 | 4.03(s,3H), 7.28(m,6H), 7.88(d,1H), 8.52(d,1H) |

TABLE IA (continued)

| Ex. No. | M.pt. (°C) | $^1$H-NMR [ppm] |
|---|---|---|
| 90 | 79 | 4.01(s,6H), 7.34(m,5H), 7.73(d,1H), 7.95(d,1H), 8.55(d,1H) |
| 91 | 86-87 | 4.00(s,6H), 7.28(m,3H), 7.52(m,3H), 7.94(d,1H), 8.55(d,1H) |
| 92 | 126-128 | 4.01(ds,6H), 7.32(m,3H), 7.84(dd,1H), 7.90(d,1H), 8.55(m,2H) |
| 93 | oil | 3.83(d,2H), 3.98(s,3H), 4.02(s,3H), 7.16(m,11H), 7.90(d,1H), 8.55(d,1H) |
| 94 | oil | 3.98(s,3H), 4.02(s,3H), 7.05(m,2H), 7.27(dd,1H), 7.45(m,2H), 7.97(d,1H), 8.58(d,1H) |
| 95 | 108 | 4.00(s,3H), 4.04(s,3H), 7.29(s,2H), 7.48(m,3H), 7.95(d,1H), 8.56(d,1H) |
| 96 | 152 | 3.81(s,3H), 4.06(s,3H), 6.69(s,1H), 7.16(s,1H), 7.38(dd,1H), 7.59(d,2H), 7.61(d,1H), 8.55(d,1H) |
| 97 | 188 | 3.90(s,3H), 4.04(s,3H), 6.85(d,1H), 7.13(s,1H), 7.58(m,4H), 8.45(d,1H) |
| 98 | oil | 2.06(s,3H), 3.76(s,3H), 4.02(s,3H), 6.83(s,1H), 7.11(s,1H), 7.32(d,1H), 8.46(d,1H) |
| 99 | oil | 3.76(s,3H), 3.96(s,3H), 6.80(s,1H), 7.10(s,1H), 7.48(m,5H), 8.47(d,1H) |
| 100 | 181-182 | 3.52(s,3H), 3.68(s,3H), 3.80(s,3H), 4.04(s,3H), 6.72(s,1H), 6.78(s,1H), 7.10(s,1H), 7.24(m,1H), 7.42(dd,1H), 7.50(d,1H), 8.45(d,1H) |
| 101 | 184 | 3.78(s,3H), 4.04(s,3H), 6.84(d,1H), 7.13(s,1H), 7.64(m,4H), 8.15(d,1H) |
| 102 | 81 | 3.70(s,3H), 3.97(s,3H), 6.64(s,1H), 7.07(s,1H), 7.50(m,4H), 7.77(d,1H), 8.42(d,1H) |

TABLE IA (continued)

| Ex. No. | M.pt. (°C) | $^1$H-NMR [ppm] |
|---|---|---|
| 103 | 143 | 3.82(s,3H), 4.05(s,3H), 6.78(s,1H), 7.12(s,1H), 7.25(s,1H), 7.48(m,1H), 7.55(m,2H), 8.45(d,1H) |
| 104 | 130 | 3.78(s,3H), 4.04(s,3H), 6.84(s,1H), 7.13(s,1H), 7.64(m,4H), 8.15(s,1H), 8.44(d,1H) |
| 105 | oil | 3.53(s,6H), 3.83(s,3H), 6.45(d,1H), 6.73(d,1H), 7.15(d,1H), 7.35(d,1H), 7.52(d,1H), 8.50(d,1H) |
| 106 | oil | 3.46(s,3H), 3.93(s,3H), 6.40(d,1H), 6.75(d,1H), 7.50(m,4H), 8.13(d,1H), 8.45(d,1H) |
| 107 | oil | 3.54(s,3H), 3.92(s,3H), 6.48(d,1H), 6.70(d,1H), 7.14(m,1H), 7.40(m,3H), 7.60(d,1H), 8.47(d,1H) |
| 108 | oil | 3.53(s,3H), 3.90(s,3H), 6.50(d,1H), 6.70(d,1H), 7.44(dd,1H), 7.50(d,1H), 8.92(d,1H) |
| 109 | oil | 1.96(s,3H), 3.46(s,3H), 3.90(s,3H), 6.40(d,1H), 6.23(d,1H), 7.18(m,4H), 7.43(d,1H), 8.44(d,1H) |
| 110 | oil | 3.46(s,3H), 3.84(s,3H), 6.48(d,1H), 6.94(m,3H), 7.32(m,1H), 7.56(d,1H), 8.33(d,1H) |
| 111 | | 0.97(t,3H), 1.48(t,3H), 1.80(m,2H), 4.20(q,2H), 4.84(t,2H), 6.62(s,1H), 7.12(s,1H), 7.37(m,3H), 7.52(d,1H), 8.46(d,1H) |
| 112 | 80-82 | 1.00(t,3H), 1.55(t,3H), 1.82(m,2H), 3.86(q,2H), 4.23(q,2H), 7.06(s,1H), 7.62(m,4H), 7.77(s,1H), 8.14(d,1H), 8.38(d,1H) |
| 113 | 145 | 1.40(t,3H), 3.98(q,2H), 4.01(s,3H), 6.65(s,1H), 7.14(s,1H), 7.36(dd,1H), 7.47(dd,2H), 7.59(d,1H), 8.50(d,1H) |

TABLE IA (continued)

| Ex. No. | M.pt. (°C) | $^1$H-NMR [ppm] |
|---|---|---|
| 114 | 178 | 3.1-3.32(m,2H), 3.66(s,3H), 3.90 (s,3H), 4.20-4.34(m,2H), 6.12(s,1H), 6.78(s,1H)), 7.28-7.61(m,3H) |
| 115 | 80-83 | 3.1-3.3(m,2H), 3.62(s,3H), 3.9(s,3H), 4.2-4.35(m,2H), 6.08(s,1H), 6.80 (s,1H), 7.46-7.7(2m,3H) |
| 116 | 166-168 | 3.24(m,2H), 3.64(s,3H), 3.93(s,3H), 4.29(m,2H), 6.10(s,1H), 6.80(s,1H), 7.35-7.48(m,3H) |
| 117 | 210-211 | 3.75(s,3H), 4.04(s,3H), 6.35(s,1H), 7.13(s,1H), 7.38-7.52(mm,3H), 7.53 (d,1H), 8.28(d,1H) |
| 118 | 110-111 | 1.0(t,3H), 1.47(t,3H), 1.70(m,3H), 3.24(m,2H), 3.75(m,2H), 4.14(q,2H), 4.30(q,2H), 6.10(s,1H), 6.78(s,1H), 7.3-7.5(m,3H) |
| 119 | oil | 1.0(t,3H), 1.54(t,3H), 1.7-1.85 (m,2H), 3.8(m,2H), 4.2(m,2H), 6.50 (s,1H), 7.13(s,1H), 7.45-7.9(m,4H), 8.27(d,1H), 8.36(d,1H) |

EXAMPLE 120

Biological activity against powdery mildew

The compounds were tested for activity against powdery mildew on cucumber, barley and wheat. The phytopathogenic fungi tested were:

| | | |
|---|---|---|
| $EG_b$: | Erysiphe graminis f.sp. hordei barley-Golden | Promise |
| $EG_W$: | Erysiphe graminis f.sp. tritici | wheat-Kormoran |
| EC : | Erysiphe cichoracearum | cucumber-Hokus |

$EG_b$ and $EG_w$:

The test plants were cultivated in 6cm diameter pots filled with Fruhstorfer Erde Typ N (Industrie-Erden-Weke Erich Archut, D-6420 Lauterbach-Wallenrod, West Germany). Seven seeds were sown in each pot. The test plants were grown for 1 week to the one-leaf stage at a temperature of 23°C during the day and 18°C at night.

The test compounds were dissolved in either acetone or methanol, depending on their solubility, to a final concentration of 5000ppm. Triton X 150 (5000ppm) was added as emulgator. The resulting mixture was diluted with deionised water to a concentration of 400ppm of active ingredient (a.i.). Two pots with seven plants each were sprayed to run off in a spray cabin with spray wash (20ml). After drying of the spray film in a hood, the plants were placed in a greenhouse at 20°-25°C.

One day after applying the test compounds to the plants, stock plants with strongly sporulating Erysiphe fungi were shaken over the test plants until the leafs were clearly visibly dusted with mildew spores. For about 3 hours, air movement was reduced as far as possible by closing doors and windows and by switching off the lighting. The plants were kept in the greenhouse until the symptoms had developed.

The assessment was carried out about 1 week after infection using the following scale:

1° = whole plant without infestation
1 = infestation up to 10% of leaf area
2 = 11-40% infestation
3 = ≦41% infestation

p denotes phytotoxicity

EC:

The test plants were sown in plant-trays and, as soon as the cotyledons were exposed, they were transferred to 6cm diameter pots filled with Fruhstorfer Erde Typ N. The test plants were grown for 1 week to treatment stage at a temperature of 23°C during the day and 18°C at night.

The test compounds were dissolved in either acetone or methanol, depending on their solubility, to a final concentration of 5000ppm. Triton X 150 (5000ppm) was added as emulgator. The resulting mixture was diluted with deionised water to a concentration of 400ppm of active ingredient (a.i.). Two pots with one plant each were sprayed to run off in a spray cabin with spray wash (20ml). After drying of the spray film in a hood, the plants were placed in a greenhouse at 20°-25°C.

One day after applying the test compounds to the plants, stock plants (variety Hoffmanns Produkta) with strongly sporulating Erysiphe fungi were treated with an air jet over the test plants until the leafs were clearly visibly dusted with mildew spores. For about 3 hours, air movement was reduced as far as possible by closing doors and windows and by switching off the lighting. The plants were kept in the greenhouse until the symptoms had developed.

The assessment was carried out about 1 week after infection using the scale described above.

The results of these tests are set out in Table II below:-

TABLE II

| Example No. | Assessment EC | $EG_b$ | $EG_w$ |
|---|---|---|---|
| 1 | 2 | 2 | 3 |
| 3 | 0 | $3^p$ | $3^p$ |
| 4 | $2^p$ | $2^p$ | $2^p$ |
| 5 | - | - | 1 |
| 8 | $3^p$ | - | - |
| 9 | $3^p$ | $3^p$ | $3^p$ |
| 10 | $3^p$ | $1^p$ | $1^p$ |
| 11 | 3 | 2 | 3 |
| 12 | 3 | $1^p$ | $1^p$ |
| 13 | 3 | 1 | 1 |
| 14 | 3 | $2^p$ | $2^p$ |
| 15 | 3 | 3 | 3 |
| 16 | 3 | 2 | 3 |
| 17 | 3 | $3^p$ | $3^p$ |
| 18 | 3 | $1^p$ | $1^p$ |
| 19 | 3 | 3 | 3 |
| 20 | $2^p$ | $3^p$ | $3^p$ |
| 21 | $3^p$ | $3^p$ | $3^p$ |
| 22 | 3 | 3 | 3 |
| 23 | 3 | 3 | 3 |
| 24 | $3^p$ | $1^p$ | $1^p$ |
| 25 | 3 | $1^p$ | $1^p$ |
| 26 | 2 | 1 | 1 |
| 27 | 3 | 1 | 1 |
| 28 | 3 | 3 | 3 |
| 33 | $3^p$ | $2^p$ | $3^p$ |
| 34 | 3 | 2 | 3 |
| 35 | 3 | $3^p$ | $3^p$ |
| 36 | 3 | $2^p$ | $2^p$ |

TABLE II (continued)

p denotes phytox

| Example No. | Assessment EC | $EG_b$ | $EG_w$ |
|---|---|---|---|
| 37 | $3^p$ | $2^p$ | $2^p$ |
| 38 | 3 | 3 | 3 |
| 39 | 3 | $3^p$ | $3^p$ |
| 40 | 3 | 2 | 3 |
| 41 | 3 | 3 | 3 |
| 42 | 3 | 3 | 3 |
| 43 | 3 | 1 | 2 |
| 44 | $3^p$ | $2^p$ | $3^p$ |
| 45 | 3 | 3 | 3 |
| 46 | 3 | 3 | 3 |
| 47 | 3 | 3 | 3 |
| 48 | 3 | 3 | 3 |
| 49 | 2 | 3 | 3 |
| 50 | $3^p$ | $3^p$ | $3^p$ |
| 51 | 3 | 2 | 3 |
| 52 | 3 | $3^p$ | $3^p$ |
| 53 | 3 | 3 | 3 |
| 58 | $1^p$ | $1^p$ | $1^p$ |
| 59 | 3 | 3 | 3 |
| 60 | $3^p$ | $3^p$ | $3^p$ |
| 61 | 3 | 3 | 3 |
| 62 | $3^p$ | $3^p$ | $3^p$ |
| 63 | 2 | $3^p$ | $3^p$ |
| 64 | 2 | $3^p$ | $3^p$ |
| 67 | $3^p$ | $3^p$ | $3^p$ |
| 70 | $1^p$ | $1^p$ | $1^p$ |
| 71 | 3 | $1^p$ | $1^p$ |
| 72 | - | $1^p$ | $1^p$ |
| 73 | 2 | $1^p$ | $1^p$ |
| 74 | $1^p$ | $1^p$ | $1^p$ |

TABLE II (continued)

p denotes phytox

| Example No. | Assessment EC | $EG_b$ | $EG_w$ |
|---|---|---|---|
| 75 | 1 | $1^p$ | $1^p$ |
| 80 | $3^p$ | $2^p$ | $3^p$ |
| 81 | $1^p$ | $3^p$ | $3^p$ |
| 82 | $2^p$ | $2^p$ | $2^p$ |
| 83 | $3^p$ | $3^p$ | $3^p$ |
| 84 | $3^p$ | $3^p$ | $3^p$ |
| 85 | $3^p$ | $3^p$ | $3^p$ |
| 86 | $1^p$ | $2^p$ | $2^p$ |
| 87 | 1 | $2^p$ | $3^p$ |
| 88 | $1^p$ | 0 | $3^p$ |
| 89 | $3^p$ | $3^p$ | $2^p$ |
| 90 | $2^p$ | $2^p$ | $2^p$ |
| 91 | $2^p$ | $3^p$ | $3^p$ |
| 92 | $2^p$ | $3^p$ | $3^p$ |
| 93 | $3^p$ | $3^p$ | $3^p$ |
| 94 | $3^p$ | $2^p$ | $2^p$ |
| 95 | 2 | 3 | 3 |
| 96 | 1 | $1^p$ | $1^p$ |
| 97 | 1 | $3^p$ | $3^p$ |
| 98 | 3 | 1 | 1 |
| 99 | $1^p$ | $1^p$ | $1^p$ |
| 100 | $3^p$ | $3^p$ | $3^p$ |
| 101 | $1^p$ | $1^p$ | $1^p$ |
| 102 | $2^p$ | $1^p$ | $1^p$ |
| 103 | $1^p$ | $1^p$ | $1^p$ |
| 104 | $1^p$ | $2^p$ | $2^p$ |
| 113 | 0 | 0 | 0 |

In further tests, the residual (R) and curative (C) activities of various compounds against powdery mildew in barley and wheat was determined at various concentrations of the test compounds. The results, expressed as % activity, are given in Table III below:-

TABLE III

| Ex. No. | | Barley | | Wheat | |
|---|---|---|---|---|---|
| | | Concentration (ppm) | Activity (%) | Concentration (ppm) | Activity (%) |
| 6 | R | 100 | 100 | 400 | 98.8 |
| | C | 100 | 100 | 400 | 68.5 |
| 7 | R | 400 | 75 | 400 | 94 |
| | C | 100 | 100 | 400 | 23 |
| 117 | R | 100 | 100 | 100 | 100 |
| | C | 100 | 100 | 400 | 100 |
| 118 | R | 100 | 100 | 100 | 100 |
| | C | 100 | 100 | 400 | 100 |
| 119 | R | 400 | 30 | 400 | 80 |
| | C | 400 | 99 | 400 | 30 |

## Claims

1. A method of combating fungus at a locus which comprises treating the locus with a compound of the general formula

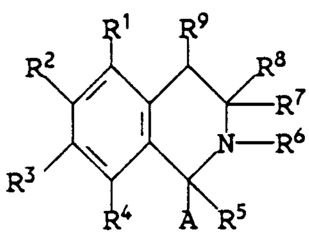

(I)

or an N-alkyl or N-phenyl halide or N-oxide thereof, in which
$R^1$, $R^2$, $R^3$ and $R^4$ independently represent a hydrogen or halogen atom, a hydroxyl group or an optionally substituted alkyl or alkoxy group, or $R^1$ and $R^2$ or $R^2$ and $R^3$ or $R^3$ and $R^4$ together with the interjacent carbon atoms represent a 5- to 7-membered saturated or unsaturated carbocyclic or heterocyclic ring in which a heterocyclic ring contains 1 to 3 heteroatoms selected from nitrogen, oxygen and sulphur atoms;
$R^5$ represents a hydrogen or halogen atom,
$R^6$ represents a hydrogen atom, or
$R^5$ and $R^6$ together represent a single carbon-carbon bond;
$R^7$ represents a hydrogen or halogen atom, an alkyl or alkoxy group optionally substituted by one or more halogen atoms, or an optionally substituted phenyl or phenoxy group;
$R^8$ and $R^9$ independently represent a hydrogen atom or together represent a single carbon-carbon bond; and
A represents an optionally substituted phenyl group.

2. A method according to claim 1 in which $R^1$, $R^2$, $R^3$ and $R^4$ independently represent a hydrogen or halogen atom, a hydroxyl group or a $C_{1-6}$ alkyl or $C_{1-6}$ alkoxy group each optionally substituted by one or more halogen atoms or $C_{1-4}$ alkoxy groups, or $R^1$ and $R^2$ or $R^2$ and $R^3$ or $R^3$ and $R^4$ together with the interjacent carbon atoms represents a 5-or 6-membered saturated heterocyclic ring containing two oxygen atoms.

3. A method according to claim 1 or claim 2 in which $R^7$ represents a hydrogen atom or a $C_{1-6}$ alkyl group.

**4.** A method according to any one of the preceding claims in which A represents a group

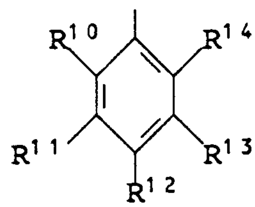

in which
$R^{10}$, $R^{11}$, $R^{12}$, $R^{13}$ and $R^{14}$ independently represent a hydrogen or halogen atom, a hydroxyl, nitro or amino group, or an optionally substituted alkyl, alkoxy, alkylamino, dialkylamino, alkylthio, alkylsulphinyl, alkylsulphonyl, aralkyl, phenyl, phenoxy or pyridyl group.

**5.** A method according to claim 4 in which $R^{10}$, $R^{11}$, $R^{12}$, $R^{13}$ and $R^{14}$ independently represent a hydrogen or halogen atom, a hydroxyl, nitro or amino group, or an alkyl, alkoxy, alkylsulphonyl, benzyl, phenyl, phenoxy or pyridyl group each optionally substituted by one or more halogen atoms.

**6.** A method according to claim 4 or claim 5 in which $R^1$, $R^2$, $R^3$ and $R^4$ independently represent a hydrogen, chlorine or bromine atom or a hydroxyl, methyl, ethyl, propyl, methoxy, ethoxy, propoxy, butoxy, pentyloxy, hexyloxy or methoxyethoxy group, or $R^2$ and $R^3$ together with the interjacent carbon atoms represent a 1,3-dioxolane ring; $R^5$ represents a hydrogen or chlorine atom, $R^6$ represents a hydrogen atom, or $R^5$ and $R^6$ together represent a single carbon-carbon bond; $R^7$ represents a hydrogen atom or an ethyl group; and $R^{10}$, $R^{11}$, $R^{12}$, $R^{13}$ and $R^{14}$ independently represent a hydrogen, fluorine, chlorine, bromine or iodine atom or a hydroxyl, nitro, amino, methyl, trifluoromethyl, methoxy, methylsulphonyl, benzyl, phenyl, phenoxy or chloropyridyl group.

**7.** The use as a fungicide of a compound of formula I, or an N-alkyl or N-phenyl halide or N-oxide thereof, as defined in any one of the preceding claims.

**8.** A compound of the general formula I as defined in claim 1 with the proviso that, when $R^1$, $R^4$, $R^5$, $R^6$, $R^7$, $R^8$ and $R^9$, all represent a hydrogen atom and A represents a 3,4,5-trimethoxyphenyl group, then $R^2$ and $R^3$ together with the interjacent carbon atoms do not represent a 1,3-dioxolane ring.

**9.** A process for the preparation of a compound of formula I as defined in claim 8 (or an N-alkyl or N-phenyl halide) or N-oxide thereof which comprises

(a) cyclising a compound of the general formula

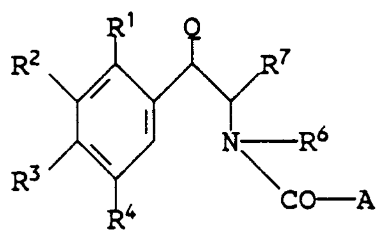

(II)

in which $R^1$, $R^2$, $R^3$, $R^4$, $R^6$, $R^7$ and A are as defined in claim 8 and, when $R^8$ and $R^9$ in the resultant compound of formula I both represent a hydrogen atom then Q represents a hydrogen atom, and, when $R^8$ and $R^9$ in the resultant compound of formula I together represent a single carbon-carbon bond then Q represents an alkoxy group, in the presence of a dehydrating agent to form a compound of formula I in which $R^5$ and $R^6$ together represent a single carbon-carbon bond;
followed, if desired, when $R^8$ and $R^9$ both represent a hydrogen atom, by
(b) hydrogenating the resulting compound of formula I to produce a compound of formula I in which $R^5$, $R^6$, $R^8$ and $R^9$ all represent a hydrogen atom; or

(c) dehydrogenating the resulting compound of formula I to produce a compound of formula I in which $R^5$ and $R^6$ together and $R^8$ and $R^9$ together both represent a single carbon-carbon bond; followed, if desired by,
(d) oxidising the compound of formula I formed in (a), (b) or (c) to form an N-oxide thereof; or
(e) converting the compound of formula I formed in (a), (b) or (c) to the corresponding N-alkyl or N-phenyl halide.

**10.** A fungicidal composition which comprises a carrier and, as active ingredient, a compound of the general formula I as defined in claim 8.

European Patent Office

# EUROPEAN SEARCH REPORT

Application Number

EP 91 20 3316

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | DE-A-3 900 233 (SANDOZ-PATENT-GMBH)<br>* Claims 1-4 *<br>--- | 8,9 | C 07 D 217/16<br>C 07 D 217/22<br>A 01 N 43/42<br>C 07 D 217/14<br>C 07 D 491/04 //<br>(C 07 D 491/04<br>C 07 D 317:00<br>C 07 D 221:00 ) |
| D,X | EP-A-0 251 361 (DUPHAR INTERNATIONAL RESEARCH B.V.)<br>* Claims 1,2 *<br>--- | 8,9 | |
| X | DE-C- 739 866 (TEMMLER-WERKE)<br>* The whole document *<br>--- | 8,9 | |
| X | US-A-3 474 104 (HANS OTT)<br>* The whole document *<br>--- | 8,9 | |
| X | FR-A-2 238 489 (G.D. SEARLE AND CO.)<br>* Page 9; example 6 *<br>--- | 8,9 | |
| X | GB-A-1 176 804 (Dr. KARL THOMAE GmbH)<br>* Claims *<br>--- | 8,9 | |
| X | GB-A-1 178 434 (F. HOFFMANN-LA ROCHE)<br>* Page 5; example 2 *<br>--- | 8,9 | **TECHNICAL FIELDS SEARCHED (Int. Cl.5)** |
| X | GB-A- 642 286 (BOOTS PURE DRUG CO., LTD)<br>* The whole document *<br>--- | 8,9 | C 07 D<br>A 01 N |
| X | US-A-3 823 148 (A.B.A. JANSEN)<br>* The whole document *<br>--- | 8,9 | |
| X | US-A-4 321 382 (C.A. LUNDBERG)<br>* Columns 5-6 *<br>--- | 8,9 | |
| X | EP-A-0 013 411 (TEIKOKU HORMONE MFG. CO., LTD)<br>* Pages 46-49 *<br>--- -/- | 8,9 | |

The present search report has been drawn up for all claims

| Place of search | Date of completion of the search | Examiner |
|---|---|---|
| THE HAGUE | 03-03-1992 | HENRY J.C. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application Number

EP 91 20 3316

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | GB-A-1 030 022 (ORGAMOL S.A.)<br>* The whole document *<br>--- | 8,9 | |
| X | GB-A- 374 627 (E. MERCK)<br>* Examples 1,2 *<br>--- | 8,9 | |
| X | FR-A- 760 825 (ASTA AG CHEMISCHE FABRIK)<br>* The whole document *<br>--- | 8,9 | |
| X | GB-A- 645 139 (SERVITA GYOGYSZERYAR ES VEGYIPARI)<br>* The whole document *<br>--- | 8,9 | |
| X | JOURNAL OF THE CHEMICAL SOCIETY, PERKIN TRANSACTIONS I, 1985, pages 275-281, Letchworth, GB; J.M. BARKER et al.: "Dehalogenation of 1-halogenothienyl-di-and -tetra-hydroisoquinolines by sodium methoxide in dimethyl sulphoxide"<br>* Page 275, compounds 1a,k,l,m; pages 278-279 *<br>--- | 8,9 | TECHNICAL FIELDS SEARCHED (Int. Cl.5) |
| X | JOURNAL OF THE CHEMICAL SOCIETY, PERKIN TRANSACTIONS I, 1976, pages 2193-2196, Letchworth, GB; A.K. BOSE et al.: "Studies on lactams. Part 45. Some carbocyclic analogues of cephalosporin"<br>* Pages 2194,2195 *<br>--- | 8,9 | |
| X | TETRAHEDRON LETTERS, vol. 26, no. 48, 1985, pages 5975-5978, Oxford, GB; C.S. HILGER et al.: "Synthesis of necatorone"<br>* The whole document *<br>--- -/- | 8,9 | |

The present search report has been drawn up for all claims

| Place of search | Date of completion of the search | Examiner |
|---|---|---|
| THE HAGUE | 03-03-1992 | HENRY J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application Number

EP 91 20 3316

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS, vol. 70, no. 7, 17th February 1969, page 306, abstract no. 28800e, Columbus, Ohio, US; B. BHATTACHARYA: "Isoquinolines derivatives. XVIII. Formation of 1-alkyl-(or alkaryl or aryl)-3-methyl-7-(or 5)-chloro isoquinolines", & INDIAN J. CHEM., vol. 6, no. 7, 1968, pages 341-345 * Abstract * --- | 8,9 | |
| D,A | EP-A-0 121 753 (HOECHST AG) * Pages 26-28; claim 1 * ----- | 1,8,9 | |
| | | | TECHNICAL FIELDS SEARCHED (Int. Cl.5) |

The present search report has been drawn up for all claims

| Place of search | Date of completion of the search | Examiner |
|---|---|---|
| THE HAGUE | 03-03-1992 | HENRY J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)